# NEW EUROPEAN PATENT SPECIFICATION

(11) **EP 1 560 517 B2**
(45) Date of publication and mention of the opposition decision: **22.04.2015**
(45) Mention of the grant of the patent: 12.08.2009
(21) Application number: 03808414.1
(22) Date of filing: 13.11.2003
(51) Int. Cl.: A61B 5/15

(54) **CAP FOR A DERMAL TISSUE LANCING DEVICE**
KAPPE FÜR LANZETTE
COUVERCLE POUR DISPOSITIF AUTOPIQUEUR DE TISSU DERMIQUE

(30) Priority: 15.11.2002 US 426683 P; 12.11.2003 US 706166
(43) Date of publication of application: 10.08.2005
(73) Proprietor: LifeScan, Inc., Milpitas, CA 95035-6312 (US)
(72) Inventor: OLSON, Lorin, Scotts Valley, CA 95066 (US); THOMSON, Anne, Inverness Inverness-shire IV3 5BY (GB); BASKEYFIELD, Damian, Edward, Haydon, Auldearn Nairn IV21 5TH (GB); LEACH, Christopher, Philip, Inverness Inverness-shire IV2 3DP (GB); DAY, Richard, Michael, Culbokie Ross-Shire IV7 8JD (GB); BOHM, Sebastian, Inverness Inverness-shire IV2 4DP (GB)
(74) Representative: Tunstall, Christopher Stephen
(86) International application number: PCT/US2003/036513
(87) International publication number: WO 2004/045375

(56) References cited:
- WO-A-01/89383
- WO-A-02/078533
- WO-A1-02/100276
- WO-A2-00/74767
- US-A- 5 951 493
- US-A1- 2002 016 606
- US-A1- 2002 082 521
- US-A1- 2003 109 777

## Description

### BACKGROUND OF THE INVENTION

1. Field of the Invention

The present invention relates, in general, to dermal tissue lancing devices and, in particular, to caps for dermal tissue lancing devices.

2. Description of the Related Art

Lancets in conventional use generally have a rigid body and a sterile needle that can be cocked and fired so as to briefly protrude from one end of the lancet. In a conventional lancing device, a lancet is mounted within a longitudinal housing and is movable along a longitudinal axis relative to the housing. Typically the lancet is spring loaded and driven along the longitudinal axis on release of the spring to puncture (i.e., "lance" or cut) dermal tissue. A blood sample can then be expressed from the punctured dermal tissue by squeezing (i.e., "milking") the finger, or other area of the body, that has been punctured for sample collection.

The lancet is used to pierce dermal tissue, thereby enabling the production of a fluid sample, typically blood, from the puncture and collection of the fluid sample for testing for an analyte, such as glucose. The blood is then transferred to a test collection device. (e.g., a test strip). This test collection device may be part of a completely separate sample collection and metering system or it may form part of a combined lancet and metering system, such as that disclosed in International Application No. PCT/GB02/03772 (published as WO 03/015627 on February 27, 2003).

. Blood samples are most commonly taken from the fingertips, where there is generally an abundant supply due to the presence of capillary blood vessels. However, the nerve density in the fingertips can cause significant pain in many patients. Sampling on alternative sites, such as earlobes, palms, limbs and the abdomen, is sometimes practiced since these alternative sites may be less sensitive. However, these alternative sites are also less likely than a fingertip to provide sufficient blood volume. Use of these alternative sites also makes blood transfer directly to test devices difficult, particularly when a combined lancet and metering device is employed.

After puncturing dermal tissue, conventional lancing devices are put to one side and the user squeezes blood from the puncture wound. This technique requires a clean storage site for the lancing device and a two-handed, two-step operation. Once at drop of blood is expressed from the lancing site, the user transfers the blood to a test strip or suitable meter.

Conventional lancing devices are available from, for example, LifeScan, Inc. of Milpitas, California, Palco Laboratories of Santa Cruz, Californian Therasense of Alameda, California and Amira Medical of Scotts Valley, California. Conventional lancing devices are described in U.S. Patent No. 5,730,753 to Morita, U.S. Patent No. 6,045,567 to Taylor et al. and U.S. Patent No. 6,071,250 to Douglas et al..

Furthermore, typical lancing devices often include a cap that engages the dermal tissue and through which the lancet protrudes on firing. The cap will, therefore, have an aperture (i.e., opening), through which the lancet will pass on firing. Typically, a distal end of the cap will be placed in contact with the dermal tissue immediately prior to firing.

When a dermal tissue lancing device with a conventional cap is placed in contact with dermal tissue, a small amount of pressure is typically applied by a user prior to launch of the lancet. This pressure forces the cap down upon the dermal tissue in a direction generally perpendicular to the surface of the dermal tissue. A small amount of dermal tissue can pass through the aperture and form a bulge, into which the lancet is launched and a puncture formed. Nevertheless, typically no blood is visible on removal of the cap and lancing device from the dermal tissue. In order to produce a blood drop that is large enough for introduction onto a test strip and subsequent measurement by a metering device, the area surrounding the puncture must be squeezed by the user.

Obtaining a blood sample in excess of 0.5 µl using a lancing needle without subsequent manipulation of the skin adjacent to the lanced cut can be problematic. To obtain larger amounts of blood, pressure (such as a pumping or milking action) is usually applied to the skin adjacent to the lanced cut, in order to force additional blood out through the cut. Some devices combine lancing and transfer to a test cell of the blood produced on lancing into an integral unit without repositioning the device. One such device is a blood glucose measuring meter that is positioned on the dermal tissue over the site to be tested. These devices do not expose the test site adequately for efficient pumping action. Vacuum at the cut has been used but is difficult to implement reliably.

This means that a user has to lay aside the metering device to squeeze the puncture area and produce blood before picking up a metering device and placing this in contact with the newly formed drop of blood.

Still needed in the field, therefore, is a cap for a dermal tissue lancing device that enables a user to obtain a fluid sample (e.g., a blood sample) without subsequent manipulation (e.g., squeezing and/or milking) of a lanced area. In addition, the cap should be compatible with use on a variety of testing sites (e.g., fingertips, limbs and abdomen). WO 01/89383 discloses a cap of the type set forth in the preamble of the accompanying claim 1. WO 02/018533 and US 2002/0016606 each disclose a cap for a dermal tissue lancing device including a sleeve member having a pair of legs for squeezing dermal tissue there between.

### SUMMARY OF THE INVENTION

Embodiments of the present invention include a cap for use with a dermal tissue lancing device that enables a user to obtain a fluid sample (e.g., a blood sample) without such subsequent manipulation as squeezing and/or milking of a lanced area. Caps in accordance with embodiments of the present invention are compatible with use on a variety of testing sites (e.g., fingertips, limbs and abdomen).

A cap according to the present invention is defined in the accompanying claim 1.

Further aspects of the invention are set out in the accompanying dependent claims.

A method for the collection of fluid sample (e.g., a blood sample from dermal tissue is also disclosed, but to which no claim is made. The method includes first providing a dermal tissue lancing device. The dermal tissue lancing device thus provided includes a housing, a lancet that is moveable with respect to the housing and a cap. The cap itself includes a proximal end for engaging with the housing, a distal end for engaging with dermal tissue and an opening (i.e., aperture) for a portion of the lancet to pass through. Furthermore, the distal end of the cap includes at least first and second resiliently deformable portions for engaging dermal tissue.

The cap of the dermal tissue lancing device is then contacted with the dermal tissue (e.g., dermal tissue of a fingertip, limb, abdomen or other site from which a fluid sample is to be collected) such that the at least first and second portions engage the dermal tissue. Subsequently, the cap is urged towards the dermal tissue (using, for example, a predetermined force) such that the at least first and second portions deform resiliently and approach theretogether. Such an approach creates a bulge in the dermal tissue by, for example, decreasing the size of the dermal tissue lancing device opening. The bulge is then lanced, using the lancet, to create a puncture in the bulge, from which a fluid sample is collected.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings in which like numerals indicate like elements, objects and forces, of which:
FIG. 1A is a perspective view of a cap for use with a dermal tissue lancing device according to an exemplary embodiment of the present invention;
FIG. 1B is a bottom (end) view of the cap of FIG. 1A;
FIG. 2A is a perspective, partially-cut-away view of the cap body illustrated in FIG. 1A in a relaxed state;
FIG. 2B is a perspective view of the cap body illustrated in FIG. 1A in a relaxed state;
FIG. 2C is a perspective, partially-cut-away view of the cap body of FIG. 1A in a compressed state as can be encountered during use (for example, when a user urges a lancet device or combined lancet and metering device incorporating such a cap against skin);
FIG. 2D is a perspective view of the cap body of FIG. 1A when compressed during use;
FIG. 3 is a perspective, partially-cut-away view of the cap body of FIG. 1A in use compressing dermal tissue illustrating a bulge 32 formed in the dermal tissue 33 under pressure applied by a user in direction 40;
FIG. 4 is a perspective, partially cut away view of the cap of FIG. 1;
FIGs. 5A and 5B show respectively, uncompressed and compressed cross-sectional views of the cap body of FIG. 1A;
FIG. 6 is a flow diagram illustrating a sequence of steps in a process for using a cap of the present invention.
FIG. 7 is a perspective cross-sectional view of a lancing device according to an embodiment of the present invention that includes a cap that is also according to an embodiments of the present invention;
FIGs. 8A and 8B depict, in cross-section, a cap according to the present invention prior to, and subsequent to, compression between two rigid surfaces.

### DETAILED DESCRIPTION OF PREFERRED EMBODIMENTS

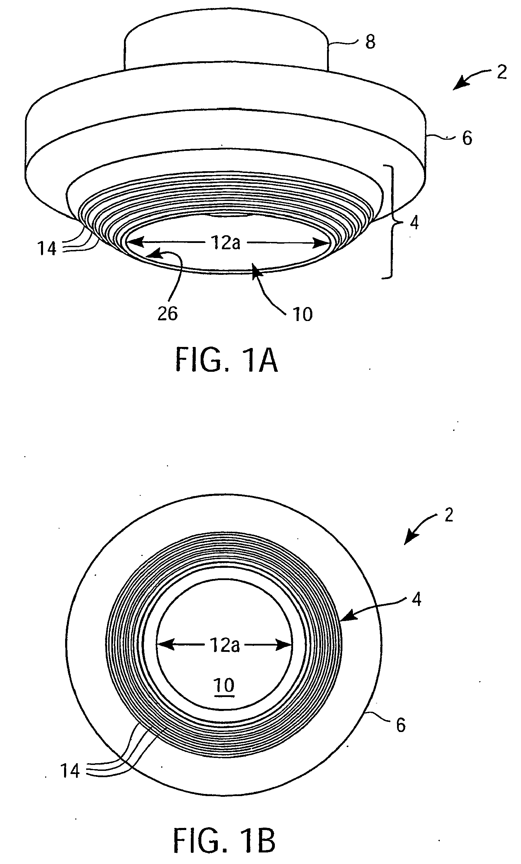

FIGs. 1A and 1B illustrate perspective and bottom (end-on) views, respectively, of a cap 2 for use with a dermal tissue lancing device (not shown) according to an exemplary embodiment of the present invention. In the embodiment of FIGs. 1A and 1B, cap 2 includes a cap body 4 (such as a flexible cap body) and retainer 6. Once apprised of the present disclosure, however, one of ordinary skill in the art can envisage alternative caps in which retainer 6 is not present. For example, a cap body of varying resilience (e.g., either of a stepped or a graded resilience) having (i) a distal end for engaging dermal tissue composed of relatively resiliently deformable material and (ii) a proximal end for engaging with a dermal tissue lancing device composed of a relatively less resiliently deformable material can be employed without a retainer. No claim is made to such a retainerless cap.

Cap body 4 includes an opening 10 (that is defined by inner edge 26 of cap body 4) and dermal tissue engaging features 14. Retainer 6 includes a stem 8. Other features of cap body 4 and retainer 6 are described below in conjunction with FIGs. 1A, 1B, 2A through 2D, 3 and 4.

Cap 2 is configured to facilitate the flow of a bodily fluid sample (e.g., a blood sample) out of a lancet cut (e.g., a puncture) in dermal tissue without such manipulation of squeezing and/or milking of the dermal tissue subsequent to lancing. In the embodiment of FIGs. 1A and 1B, cap body 4 is formed of an elastomeric material. During use, cap body 4 encircles an area of the dermal tissue that is to be lanced through opening 10 (also referred to as an aperture) in the cap body. The diameter of opening 10 when cap body 4 is in a relaxed state is denoted as distance 12a in FIG. 1A.

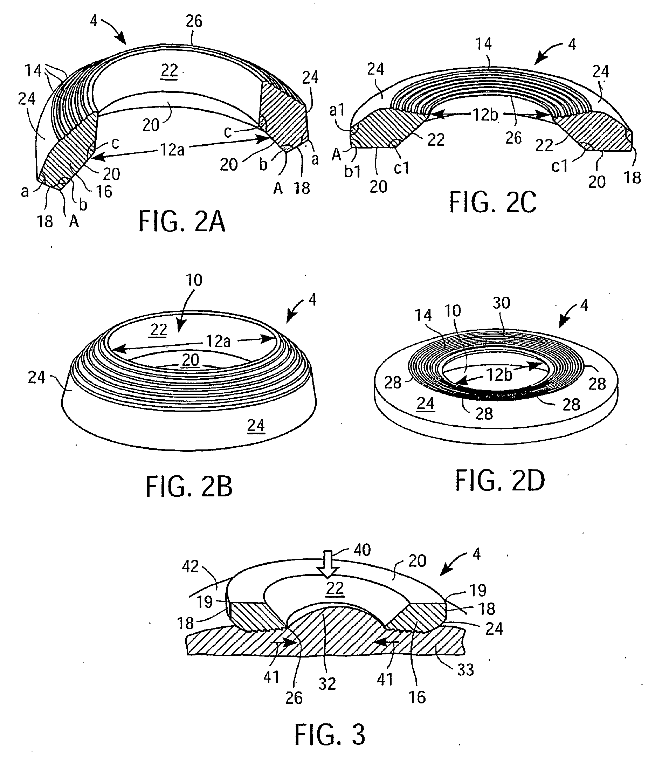

In the embodiment of FIGs. 1A and 1B, inner edge 26 defines a circular opening 10, however, the shape of opening 10 can be any suitable shape including, but not limited to, a square shape, a triangular shape, a C-shape, a U-shape (thus allowing access to opening 10 from the side by, for example, a test strip to enable *in-situ* transfer of a blood sample to the test strip), an hexagonal shape and an octagonal shape.
Furthermore, cap body 4 can have a plurality of resiliently deformable segments (i.e., portions), not limiting to two, the distal ends of which engage the skin and, on compression, these segments can resiliently deform urging the dermal tissue into a bulge. Preferably, but not necessarily, these segments resiliently deform and approach theretogether in a synchronized way to create a bulge in the dermal tissue. The embodiment of FIGs. 1A and 2A, however, has an opening 10 with a continuous inner edge 26 through which skin can pass, as well as dermal tissue engaging features 14 for engaging the skin and urging it into a bulge upon compression of cap body 4 of cap 2.

Retainer 6 includes an optional stem 8 for connecting cap 2 to a housing (not shown) of a dermal tissue lancing device. Stem 8 may, if desired, be threaded to mate with a corresponding thread on the housing. Alternative connecting mechanisms may be used, including, but not limited to, a snap fit connection and a telescoping connection. Indeed, stem 8 may be fixably connected to the housing, although a degree of controlled movement of cap 2 with respect to the housing along the direction of lancing will allow control of the position of the opening 10 in cap body 4 with respect to the lancet in a rest position.

In the embodiment of FIGs. 1A and 1B, cap body 4 is formed of an elastomeric material and is readily resiliently deformable for insertion into retainer 6 and use. Thus, a user can squeeze sides (e.g., base surface 18) of the cap body together and slip the thus deformed cap body into the retainer, whereafter the cap body returns to an essentially undeformed shape and is ready for use. In the embodiment of FIGs. 1A and1 B, cap body 4 is rotatably snug fit within retainer 6 (as discussed in detail below).

Cap body 4 includes dermal tissue engaging features 14 in the form of a plurality of concentric protruding ridges that surround opening 10. Alternative dermal tissue engaging features, such as a roughened surface, other forms of protrusion, recesses, etc., can be envisaged by those skilled in the art once they are apprised of the present disclosure.

FIGs. 2A and 2B depict a cross-sectional perspective and perspective views, respectively, of cap body 4 in an initial relaxed, non-deformed, uncompressed state. Alternatively, embodiments can be envisaged by those skilled in the art in which the initial (i.e., prior to use) state of the cap 2 is partially compressed (e.g., partially compressed within the confines of retainer 6). A cross-section 16 through cap body 4 is, for example, generally squat, and thus the height of the cross-section is generally of the same order of dimension as the width of the cross-section. The distal end of cylindrically shaped internal surface 22 of cap body 4 defines inner edge 26 of opening 10. A second internal surface 20 of cap body 4 is frusto-conical in shape and adjoins internal surface 22 at its proximal end and base surface 18 of cap 4 at its distal end. Base surface 18 meets outer surface 24, which is of an approximately cylindrical shape.

Opening 10 of cap 2 is typically circular. Alternative suitable opening shapes, such as square, triangular, rectangular, hexagonal, octagonal or any suitable shape for engaging skin can be envisaged by those skilled in the art, as can the use of a cap that includes separate segments (i.e., separate portions) or fingers, the ends of which essentially define at least a part of the opening and that resiliently deform under pressure to urge the formation of a target site bulge for lancing within the opening.

FIGs. 2C and 2D illustrate cap body 4 in an activated (i.e., compressed) state. Once cap body 4 is partially or wholly compressed (due to, for example, contact with and urging against dermal tissue [i.e., skin] during lancing use), the diameter 12b of opening 10, that is a reduced diameter relative to the diameter 12a of cap body 4 in a relaxed or partially relaxed state, is obtained. Similarly the diameter of dermal tissue engaging features 14 can be reduced on compression of cap body 4.

Referring to FIGs. 1A, 1B, 2A through 2D, 3 and 4, during lancing, a user presses the lancing device and, hence, cap 2, onto the skin (i.e., onto a dermal tissue target site, also referred to as a testing site). Such pressing applies a force to resiliently deformable cap body 4 that is substantially perpendicular to the dermal tissue (i.e., to a plane containing opening 10), resulting in opening 10 being adjacent with the surface of the skin. This force deforms (e.g., compresses) cap body 4, causing inner edge 26 and dermal tissue engaging features 14 to move radially inwards, thus reducing the size of the opening to dimension 12b. A bulge is formed in the skin encircled by the reduced opening and, following lancing of the bulge, bodily fluid (e.g., blood) emerges from the puncture without such manipulation as squeezing and/or milking of the lanced area. To increase the amount of blood produced, the application of the force on the skin by the lancet device/cap can be maintained for a predetermined time period after lance (i.e., post-lance pressure). The longer the post-lance pressure time period, the more blood is generally produced. The amount of blood produced can be further increased by also applying and maintaining such force prior to lance (i.e., pre-lance pressure) for a predetermined time period. One skilled in the art can optimize production of blood by experimenting various combination of pre-lance pressure and/or post-lance pressure time duration. Preferred post-lance pressure time duration for producing sufficient amount of blood to be used for analyte testing (e.g., glucose concentration testing) is approximately 5 seconds. Caps according to the present invention are particularly useful in combined lancet and metering devices for measuring an analyte such as glucose. More particularly, caps according to the present invention are useful for such devices which measure an analyte *in-situ,* since there is no need to reposition the device, following lancing, for squeezing and/or milking the lanced area for production of a sample.

Movement of inner edge 26 and dermal tissue engaging features 14 radially inwards causes skin, blood and sub-dermal tissue beneath the skin into a bulge within the circumference of the opening (i.e., aperture). Such movement is speculated to result in an increased (relatively high) pressure within the bulge.

On compression, cap body 4 pivots inwards about circular ridge point A so that second internal surface 20 becomes a base (i.e., the proximal end of cap body 4 closest to the lancing device). Second internal surface 20 changes from a frusto-conical shape into a disc-like shape. Likewise, internal surface 22 loses its cylindrical shape and becomes frusto-conical in shape. The diameter 12b of opening 10 reduces compared to diameter 12a (i.e., portions of inner edge 26 and dermal tissue engaging features 14 approach one another as the opening reduces in size).

It is speculated without being bound that the following discussion provides an explanation of the effect of caps according to the present invention on lancing. The force applied by a user in a direction generally perpendicular to a plane of opening 10 is translated into a force acting radially inwardly on the dermal tissue by the action of the retainer 6 on the cap body 4. This can be seen more clearly in FIG. 3, in which the force in direction 40 (indicated by the open arrow of FIG. 3) causes radially inwardly directed force 41 (indicated by the closed arrows in FIG. 3) to act upon the dermal tissue, thereby forming the dermal tissue into a bulge 32.

Typically, force 41 can result from the reaction of the cap body 4 acting radially outward on the retainer 6 during its resilient deformation. The term "resiliently deformable" means that on release from an external force (e.g., force 41), the cap body at least partially and in some embodiments wholly resumes its original shape (optionally within the confines of a retainer in the circumstance that such a retainer is provided). For the purpose of explanation only, retainer 6 is illustrated as circular and continuous in FIGs. 1A and 1B. One skilled in the art will recognize that retainer 6 may be of any suitable shape or configuration, including but is not limited to, square, triangular and hexagonal, or it may be formed as discrete segments defining such shapes. Sub-dermal material and blood are also drawn into the bulge. Surface tension in the dermal tissue within the bulge 32 imparts pressure to the bulge. In addition, the provision of an inner edge 26 provides a pinch point, or more precisely a pinch ring, at inner edge 26, thereby providing a local increase in pressure and restricting blood flow out of bulge 32.

The provision of a distinct sharply curved inwardly facing inner edge 26 on opening 10 is speculated to enable pressure from the resiliently deformed cap to be exerted directly onto the skin that has passed through the opening. This urges the formation of a bulge and constricting of the dermal tissue as blood and sub-dermal tissue are forced into the bulge whilst limiting return slippage of the skin past the internal edge. Furthermore, once blood and sub-dermal tissue are forced into the bulge within the opening, portions of the inner edge are believed to provide a higher pressure region and/or pinch points limiting the ability of the blood and sub-dermal tissue below the skin to exit the bulge despite the exertion of pressure to do so from surface tension caused by the constriction of the skin forming the bulge. It is further speculated without being bound that compression of the cap body 4 occurs in two stages. In an initial stage, the cap body 4 serves to apply a ring of pressure approximately perpendicular to the skin surface that constricts the flow of blood into and out of the ringed area. In a second stage, the diameter of the inner ring in contact with the skin decreases so that blood that is already constrained by the cap body is brought further into the center of the ringed area and compressed.

Bulge 32 can be centered within the target area of the lancing needle. Furthermore, due to the presence of a bulge, when a lancet (e.g., a needle-type lancet) punctures the dermal tissue, the resulting blood flow is larger than from the lanced cut made by a conventional lancing device without using caps according to embodiments of the present invention.

Materials other than elastomeric materials can be used to form a cap according to embodiments of the present invention as long as, for example, a force essentially perpendicular to the dermal tissue results in deflection of the material and hence deflection of the skin (i.e., dermal tissue) in such a way as to force the blood beneath the skin into a bulge. For example, a cap body 4 can be made of deformable polystyrene. Thus, on initial compression, such a cap would compress the skin to form a bulge as herein described, but further compression could result in permanent deformation of the cap body. Thus the cap body is prevented from being re-used more than once. This can be an advantage by reducing the risk of infection from a cap becoming contaminated with body fluid during a first lancing operation and then being re-used for a later lancing operation. The following description is given by way of speculative explanation but is not intended to be limiting.

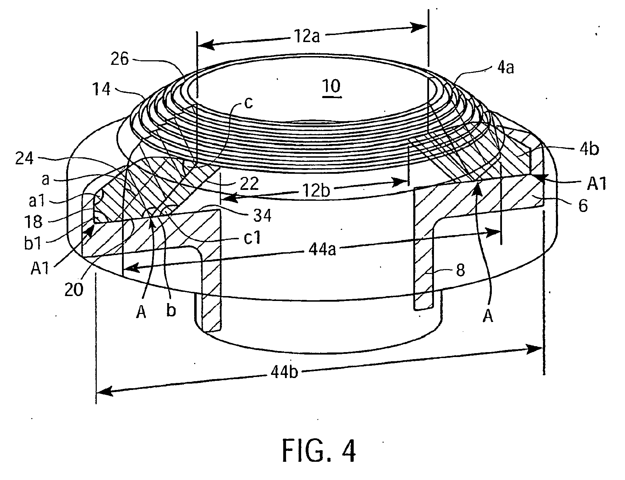

Rotation or pivoting of cap body 4 can be seen in FIG. 4 in which cap body 4a is in a relaxed state and cap body 4b is shown in a compressed state. On compression of cap body 4a into position 4b, angles a, b and c become angles a1, b1 and c1, respectively. In addition, pivot A not only acts as a pivot, but also moves radially outwards to form an outer corner of newly formed disc-shaped second internal surface 20. Thus, the region of the cap body immediately outside the opening is pushed toward the center of the opening pulling the dermal tissue within it. The external diameter of the base region of cap body 4 expands from 44a in relaxed state to 44b in a compressed state.

It can be seen in this diagram that diameter 12a of opening 10 in a relaxed state is greater than diameter 12b of opening 10 in a compressed state. It can also be seen in FIG. 4 that when cap body 4 is in a relaxed state, it is free to rotate within a recess provided in retainer 6. This means that cap body 4 can be readily inserted into retainer 6 ready for use, or removed for cleaning, by simply deforming (e.g., by pinching) and sliding cap body 4 in and out of retainer 6.

A stop surface 34 (also referred to simply as a "stop") is provided to limit the compression of cap body 4 in a longitudinal direction (i.e., generally perpendicular to a plane containing opening 10) with respect to the lancing device. Typically the lance also extends and retracts along this direction, although it may act at an angle to this direction.

Within a lancing device, the use of a stop enables a lancet rest and cocked position to be determined with respect to the stop and therefore with respect to a bulge of dermal tissue of nominal size protruding within the opening. Therefore, it is possible to determine or estimate for a nominal bulge size the depth a lancet will penetrate on launch, as long as the cap body is deformed sufficiently to have reached the stop position. Thus, by controlling the location of the lance with respect to the stop and/or the cap with respect to the stop, the depth of penetration of the lance into the dermal tissue can be controlled. By trial and error, the user will be able to determine for each particular location about the body what settings of lance with respect to stop and/or cap with respect to stop is required to produce a suitably sized drop of blood with the least or an acceptable amount of discomfort.

Furthermore, it will be appreciated by those skilled in the art that as a user pushes (presses) cap body 4 down onto the dermal tissue, the base region of cap body 4 (closest to the lancing device) will travel radially outwards and could be limited by the inner surface of retainer 6. Thus, it is speculated without being bound that retainer 6 may provide a reaction force so that further compression of cap body 4 causes radially inward travel of inner edge 26 of opening 10, rather than radially outward travel of pivoting ring A. This particular form of cap body is especially suitable for relatively hard dermal tissue such as the fingers, although it can be used elsewhere on a body.

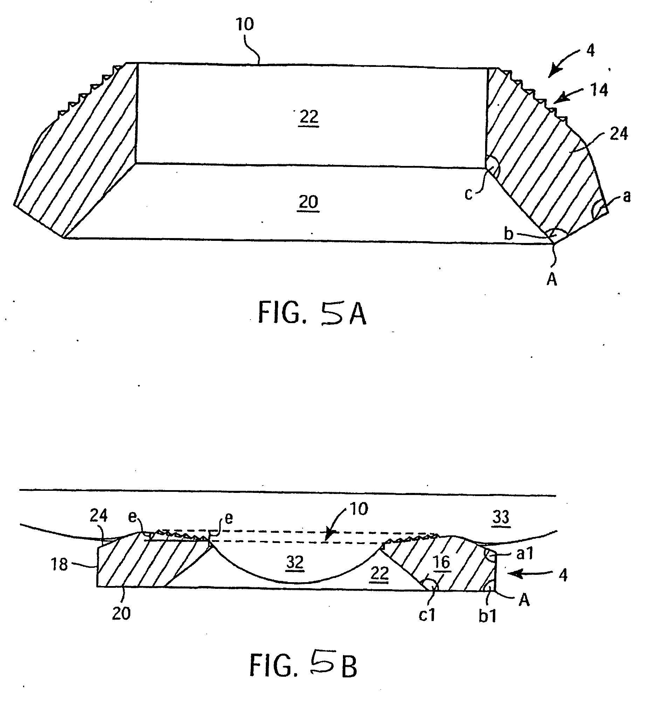

Figures 5A and 5B illustrate in more detail the deformation induced in cap body 4 during use. Second internal surface 20 is pushed flat against the base of the retainer ring (not shown) to form a disc shape. In other words, the base portion of the cap body 4 splays out whilst the upper portion closest to the skin moves radially inwards. Indeed, this radially inward motion and rotation of the cap body about pivot A can, for certain cross-sectional shapes of cap body 4, cause the formation of a recess immediately adjacent bulge 32. Thus, the portion of the cap body surface containing dermal tissue engaging features 14 is now at an angle "e" to a plane containing opening 10.

**Example 1**

Comparison of Performance of a Conventional Rigid Cap with a Cap According to the Present Invention (referred to as a "Soft Cap"):

A comparative study between a conventional rigid cap (i.e., rigid cap #1) and a cap according to the present invention (referred to as a "Soft Cap") was conducted using a 30 gauge lancet (i.e., an Ultra-Fine II lancet™) available from Beckton Dickinson of Franklin Lakes, NJ. As can be seen from the results listed in Table 1, the average amount of blood produced in the comparative study was significantly greater using a Soft Cap than the conventional cap. The minimum amount of blood produced using a Soft Cap was 0.8 µl, an amount that was significantly greater than that produced by the conventional rigid cap. The overall success rate in therms of the percentage number of lancing events giving greater than 1 µl of blood (typically the minimum volume required to give an accurate assessment of an analyte, such as glucose, in blood) is 92% for the Soft Cap compared to 25% with the conventional rigid caps

**Table 1: BLOOD VOLUME (µl)**

| Cap | Average (µl) | Minimum (µl) | Maximum (µl) | Success Rate = % Number of sticks giving>1µl blood | |
|---|---|---|---|---|---|
| Rigid Cap #1 | 0.5 | 0.1 | 1.3 | 25% | BD |
| Soft Cap | 4.3 | 0.8 | 6.0 | 92% | Lancet |

Table 2 below illustrates the results of a comparative study that was conducted in a similar manner to that described above bit that employed an alternative conventional lancet with a second type of conventional rigid cap (i.e., rigid cap #2).

**Table 2: BLOOD VOLUME (µl)**

| Cap | Average (µl) | Minimum (µl) | Maximum (µl) | Success Rate = % Number of sticks giving >1µl blood |
|---|---|---|---|---|
| Rigid cap #2 | 0.4 | 0.1 | 0.7 | 0% |
| Soft Cap. | 3.3 | 1.3 | 6.3 | 100 |

**Example 2**

Study of Caps According to the Present Invention Formed of Various Materials

Four different cap materials were tested with the embodiment shown in FIGs. 1 to 4, (device L1) and the results are shown in table 3. Two types of silicone (of hardness 40A and 60A on the Shore Index) and two types of polyurethane (30A and 50A in the Shore Index) were used. Blood volume obtained upon lancing was measured and the pain was assessed for 32 participants. Each participant received two finger sticks with each cap design on the same finger of both hands for a total of eight finger sticks per participant. The blood volume was measured with calibrated capillary pipettes and the pain scale is given below table 3.

Success was defined as obtaining more than 1 µl of blood. As is shown in Table 3, blood volume with each type of cap was greater than 1 µl. Pain was barely noticeable with each material. Use of all but the silicone 60A resulted in a success rate greater than 90%. The greatest blood volume, highest pain rating and highest success rate was obtained with the silicone 40A cap. It will thus be appreciated by those skilled in the art that a variety of materials which are resiliently deformable can be used in caps according to the present invention.

**Table 3: Data Comparing cap Material**

| Cap material | Average Blood Volume (ul) | Average Pain (0-10) | Success Rate |
|---|---|---|---|
| Silicone 60A | 2.77 | 2.24 | 78.13 |
| Polyurethane 30A | 3.25 | 2.47 | 93.75 |
| Silicone 40A | 3.54 | 2.72 | 95.32 |
| Polyurethane 50A | 3.09 | 2.40 | 93.75 |

| | | | |
|---|---|---|---|
| Pain Scale: 0 = could not feel 2 = barely noticeable 4 = slightly painful 6 = somewhat painful 8 = painful 10 = very painful | | | |

As will be appreciated by those skilled in the art, caps, lancet devices incorporating a cap and combined lancet and metering devices incorporating caps according to the present invention greatly facilitate the production of a fluid sample (e.g., a blood sample) at a puncture (lancing) site without requiring a subsequent squeezing/milking action. This facilitates in-situ testing of a fluid sample by means of a fluid collection device (such as a test strip) that is introduced at the puncture site just after a lancet has been retracted. Such a device allows the user to easily undertake two actions (i.e., placing a device over a suitable portion of dermal tissue and launching the device), thereby simplifying the collection of a sample and rending it more convenient for a user.

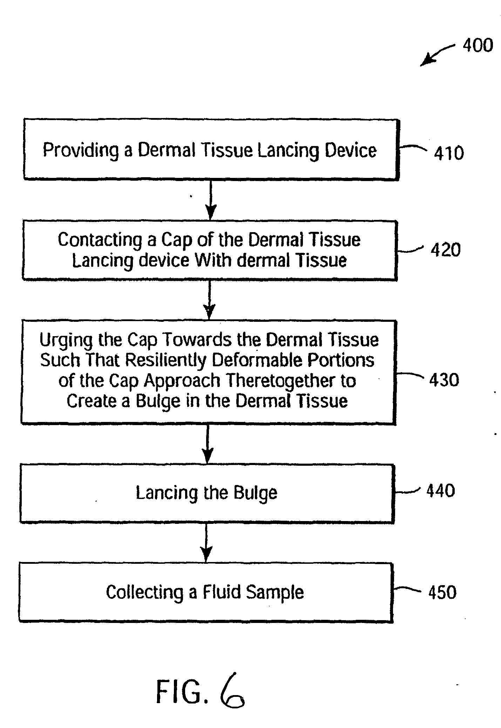

Referring to FIG. 6, a method 400 for the collection of a fluid sample (e.g., a blood sample) from dermal tissue according to an exemplary embodiment of the present invention includes providing a dermal tissue lancing device, as set forth in step 410. The provided dermal tissue lancing device includes a housing, a lancet that is moveable with respect to the housing and a cap. The cap includes a proximal end for engaging with the housing, a distal end for engaging with dermal tissue, and an opening (i.e., aperture) for a portion of the lancet to pass through. The distal end of the cap includes at least first and second resiliently deformable portions for engaging dermal tissue.

Next, as set forth in step 420, the cap of the dermal tissue lancing device is contacted with the dermal tissue (e.g., dermal tissue of a fingertip, limb, abdomen or other site from which a fluid sample is to be collected) such that the at least first and second portions engage the dermal tissue.

The cap is then urged towards the dermal tissue such that the at least first and second portions deform resiliently and approach theretogether, as set forth in step 430. The approaching of the first and second portions, which may or may not occur in a synchronized fashion, creates a bulge in the dermal tissue. The urging together of the first and second portions can create the bulge by, for example, decreasing the size of the dermal tissue lancing device opening. Optionally, the cap can be held for a predetermined time period in such a way that a bulge is maintained in the dermal tissue (i.e., pre-lance pressure).

The bulge is then lanced, using the lancet, to create a puncture in the bulge, as set forth in step 440. Optionally, the cap can be held for a predetermined time period in such a way that a bulge is maintained in the dermal tissue subsequent to the lance (i.e., post-lance pressure). Preferred time duration for the post-lance pressure is approximately 5 seconds. A fluid sample is then collected from the puncture, as set forth in step 450. One skilled in the art will recognize that method 400 can be modified to employ any of cap, lancing device or combined lancing and metering device according to the present invention.

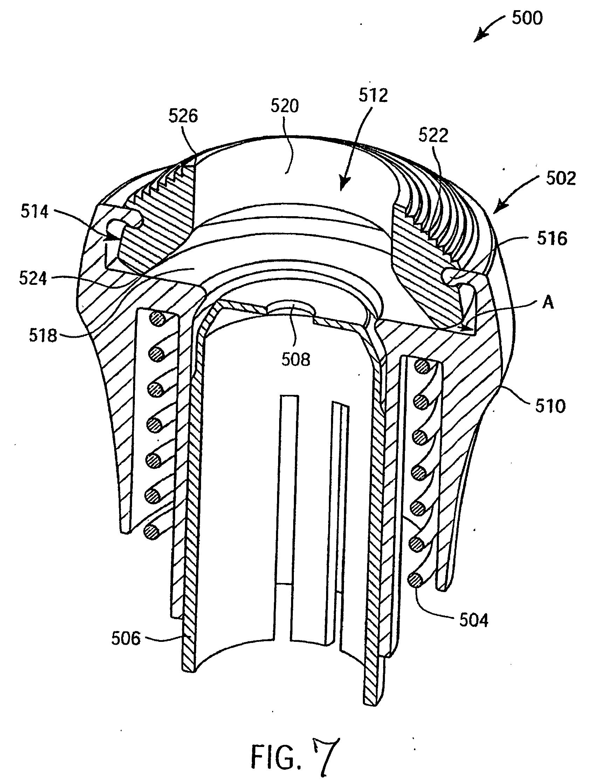

FIG. 7 is a cross-section schematic view of a lancing device 500 according to an exemplary embodiment of the present invention. Lancing device 500 includes a cap 502 (also according to an embodiment of the present invention), a spring 504 and a floating probe 506. Floating probe 506 includes an aperture (i.e., opening) 508 configured for a lancet (not shown) to pass therethrough. Floating probes, such as floating probe 506 of FIG. 7, are described in co-pending U.S. Patent Application No. 10/690,083.

Cap 502 includes a retainer 510 and a flexible cap body 512. Retainer 510 has an inwardly facing recess 514 for receiving flexible cap body 512. Retainer 510 also has an inwardly protruding rim 516, and a base surface 518.

Flexible cap body 512 has an opening 520 configured to allow a lancet (not shown) to pass therethrough, an outer surface (rebate) 522, a lower rim 524 and an upper rim 526.

Retainer 510 rests on spring 504, which in turn rests on an upper housing (not shown) of lancing device 500. Inwardly protruding rim 516 of retainer 510 is configured to operatively cooperate (as described below) with outer surface 522 of flexible cap body 512. Lower rim 524 of flexible cap body 512 provides for contact between the flexible cap body 512 and base surface 518 of retainer 510.

During use of lancing device 500, upper rim 526 (also referred to as the distale end) of flexible cap body 512 is pushed against a target site (e.g., dermal tissue) by movement of the lancing device towards the target site, causing flexible main cap body 512 to compress and rotate about protruding rim 516 of retainer 510. Lower rim 524 (also referred to as the proximal end) of flexible cap body 512 slides outwards along base surface 518 of retainer 510. Meanwhile, upper rim 526 grips (or slides then grips) the target site, causing the target site to bulge. During compression, outer surface 522 travels over, and pivots with respect to, protruding rim 516 of retainer 510. In addition, during use, flexible cap body 512 travels within inwardly facing recess 514 in the direction of arrow "A".

From the foregoing description, one skilled in the art will recognize that flexible cap body 512 of lancing device 500 can be considered as including two portions (i.e., first and second symmetric portions with one of the portions being depicted in FIG. 7) that form a continuous ring adapted for engaging and surrounding dermal tissue.

During use, there is a potential for caps to come into contact with blood or other bodily fluid. Such contact can conceivably lead to contamination of the cap with undesirable micro-organisms (e.g., bacteria or fungi). Therefore, it can be beneficial for caps to be formed, at least partially, of an anti-microbial material, anti-fungal material and/or anti-viral material, for example, anti-microbial plastic, anti-microbial resin and/or anti-microbial silicone. Suitable anti-microbial materials include anti-microbial compounds that include trichloro-phenol group, such as 2,4,4 -trichloro -2-hydivxy diphenol ether. The anti-microbial compound can be, for example, a coating of the cap or incorporated directly in the cap.

**Example 3**

Theoretical Mechanical Analyses of an Exemplary Cap

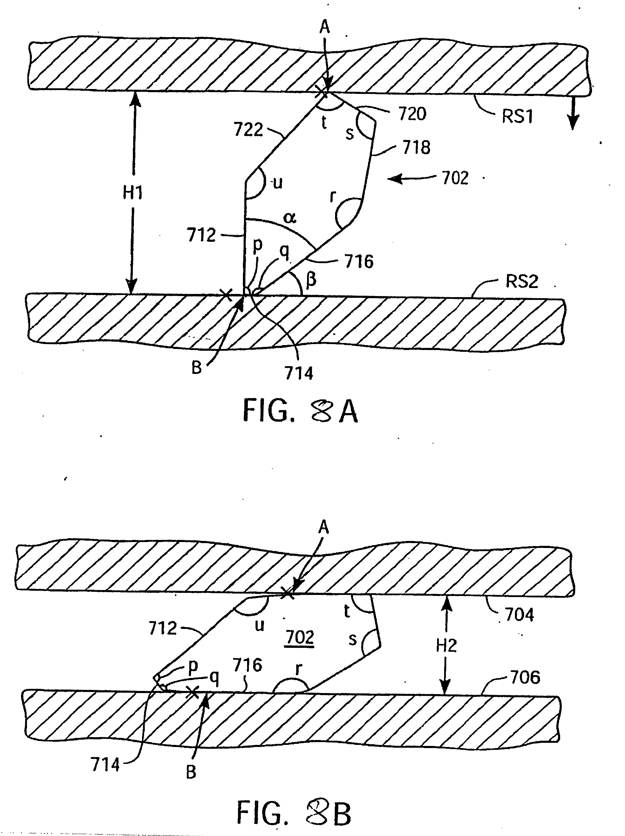

Insight into the use (i.e., compressive operation) of caps according to embodiments of the present invention was obtained through a theoretical mechanical analysis of an exemplary cap configuration. FIGS. 8A and 8B show a cross-section through the cap 702 prior to and after compression, respectively, by opposing rigid surfaces RS and RS2. In this analysis, the cross-section of the exemplary cap is six-sided. Initial points of contact between cap 702. and RS1 and RS2 are labeled "A" and "B" respectively. Furthermore, the initial distance between RS1 and RS2 is labeled H1 (see FIG. 8A), while the compressed distance is H2 (see FIG. 8B).

Cap 702 has six sides, 712, 714, 716, 718, 720 and 722. In addition, the internal angles of the cross section of cap 702 are shown as angles p, q, r, s, t and u. Furthermore, the angle between sides 712 and 716 is angle α, while the angle between side 716 and RS2 is angle β. Angle α and angle β determine the final position of cap 702 after compression. For example, a relatively small angle α can result in cap 702 moving further from point B upon compression than would be the case with a relatively large angle α.

In the positioning of FIG. 8B, the geometry of cap 702 prevents further rotation. Any further increase in force will act to compress cap 702 rather than encourage further rotation.

Since caps are generally not compressed between two parallel rigid plates during use, one skilled in the art will recognize the above analysis, and related FIGs. 8A and 8B, is presented for descriptive purposes only.

It should be understood that various alternatives to the embodiments of the invention described herein may be employed in practicing the invention. It is intended that the following claims define the scope of the invention.

## Claims

1. A cap (2) for a dermal tissue lancing device (202), the dermal tissue lancing device including a housing (208, 210) and a lancet (200) that is movable with respect to the housing, the cap comprising:
a cap body (4);
a retainer (6); and
an opening (10) for a portion of the lancet (200) to pass through;
wherein the cap body (4) has a proximal end for engaging with the retainer (6) and a distal end for engaging with dermal tissue (33);
wherein the retainer (6) has a proximal end for engaging with the housing (208, 210) and a distal end for engaging with the cap body (4);
wherein the distal end of the cap body (4) includes at least a first portion and a second portion for engaging dermal tissue, the at least first and second portions being resiliently deformable such that when, in use, the cap (2) contacts and is urged towards dermal tissue (33), the at least first and second portions deform resiliently, engage the dermal tissue and approach theretogether;
**characterised in that** a cross-sectional shape (16) of the cap (2) is such that a pivot (A) is formed on the cap body (4) and within the retainer (6) such that when the cap, including the cap body and retainer, is urged, in use, towards dermal tissue (33), the cap body (4) pivots within the retainer (6) such that the size of the opening (10) is reduced.

2. The cap of claim 1, wherein the at least first and second portions are adapted such that when the cap (2) contacts and is urged toward dermal tissue (33), the at least first and second portions deform resiliently such that the opening (10) is reduced in size.

3. The cap of claim 1 or claim 2 in which at least one of the first portion and second portion includes an edge portion (26) that serves to define a part of the opening (10).

4. The cap of any one of claims 1 to 3 in which the at least first and second portions form a continuous ring adapted for engaging and surrounding dermal tissue (33).

5. The cap of claim 4, wherein the continuous ring is substantially circular.

6. The cap of any one of claims 1 to 5, wherein the cap (2) is at least partially formed of an anti-microbial material.

7. The cap of any one of claims 1 to 5, wherein the cap (2) includes a coating of anti-microbial material.

8. The cap of claim 6 or claim 7, wherein the anti-microbial material includes a trichlorophenol compound, such as 2,4,4,-trichloro-2-hydroxy-diphenol.

9. The cap of any one of claims 1 to 8, wherein the at least first and second portions are inwardly bendable portions.

10. The cap of claim 9, wherein the inwardly bendable portions include skin-gripping teeth (14).

11. The cap of any one of claims 1 to 10, wherein the at least first and second portions have a six-sided cross-section (16).

12. The cap of claim 11, wherein the six-sided cross-section (16) includes an internal angle (α) that determines a compressed position of the cap (2) during use.

13. The cap of any one of claims 1 to 12, wherein the cap (2) is configured to be stackable with other identical caps.

14. The cap of any one of claims 1 to 13, wherein the at least first and second portions deform resiliently, engage the dermal tissue (33) and approach theretogether such that a bulge (32) is created in the dermal tissue.

15. The cap of claim 14, wherein the cap (2) is configured for applying a pre-lance pressure to maintain the bulge (32) prior to lancing by the dermal tissue lancing device (202) and for applying a post-lance pressure to further maintain the bulge after lancing by the dermal tissue lancing device.

16. The cap of any preceding claim, wherein the proximal end of the cap body (4) is prevented by the retainer (6) from deforming outwardly in a plane substantially parallel to a plane containing the opening (10).

17. The cap of any preceding claim, wherein the proximal end of the cap body (4) freely rests within the retainer (6).

18. The cap of any one of claims 1 to 17, wherein the retainer (6) includes a stop (34) for preventing deformation of the cap body (4) in a direction generally perpendicular to a plane containing the opening (10).

19. The cap of any one of claims 1 to 18, wherein the retainer (6) has an inwardly facing recess for receiving the cap body (4) and an inwardly protruding rim configured to operatively cooperate with an the outer surface of the cap body.

20. The cap of any one of claims 1 to 19, wherein the cap body (4) is a flexible cap body.

21. A lancet device (202) comprising:
a housing (208, 210);
a lancet (200) that is moveable with respect to the housing; and
a cap (2) according to any one of claims 1 to 19.

22. A combined lancet and metering device for lancing dermal tissue (33), extracting a fluid sample and measuring an analyte within said fluid sample, the combined lancet and metering device comprising:
a lancet device (202) according to claim 21 and a metering device for measuring the analyte within the fluid sample.

23. The combined lancet and metering device of claim 22 in which a fluid collection device is delivered to a position adjacent a lancing position after lancing has occurred such that the fluid sample can be collected and measured within the metering device without repositioning of the combined lancet and metering device with respect to the puncture.

24. The combined lancet and metering device of claim 23 wherein the fluid collection device is a test strip.

25. The device of any one of claims 1 to 24, for use in the collection of a fluid sample from dermal tissue (33).

## Patentansprüche

1. Kappe (2) für eine Vorrichtung (202) zum Durchstechen von Hautgewebe, wobei die Vorrichtung (202) zum Durchstechen von Hautgewebe ein Gehäuse (208, 210) und eine Lanzette (200), die in Bezug auf das Gehäuse bewegbar ist, umfasst, wobei die Kappe aufweist:
ein Kappengehäuse (4);
eine Halterung (6); und
eine Öffnung (10), durch die ein Teil der Lanzette (200) treten kann;
wobei das Kappengehäuse (4) ein proximales Ende zum Eingriff mit der Halterung (6) und ein distales Ende zum Eingriff mit Hautgewebe (33) hat; wobei die Halterung (6) ein proximales Ende für den Eingriff mit dem Gehäuse (208, 210) und ein distales Ende für den Eingriff mit dem Kappengehäuse (4) hat; wobei das distale Ende des Kappengehäuses (4) wenigstens einen ersten Bereich und einen zweiten Bereich zum Eingriff mit Hautgewebe aufweist, wobei der wenigstens erste und zweite Bereich federnd deformierbar sind, so dass, wenn beim Einsatz die Kappe (2) Hautgewebe (33) berührt und auf dieses zu gezwungen wird, sich der wenigstens erste und zweite Bereich federnd deformieren, das Hautgewebe greifen und sich annähern; **dadurch gekennzeichnet, dass** eine Querschnittsform (16) der Kappe (2) derart ist, dass eine Schwenklinie (A) auf dem Kappengehäuse (4) und innerhalb der Halterung (6) gebildet wird, so dass, wenn die Kappe, einschließlich dem Kappengehäuse und der Halterung, beim Einsatz auf Hautgewebe (33) zu gezwungen wird, das Kappengehäuse (4) innerhalb der Halterung (6) derart verschwenkt, dass die Größe der Öffnung (10) verringert wird.

2. Kappe nach Anspruch 1, bei der der wenigstens erste und zweite Bereich derart ausgelegt sind, dass, wenn die Kappe (2) Hautgewebe (33) berührt und auf dieses zu gezwungen wird, sich der wenigstens erste und zweite Bereich federnd deformieren, so dass die Öffnung (10) in ihrer Größe verringert wird.

3. Kappe nach Anspruch 1 oder Anspruch 2, bei der wenigstens einer, der erste Bereich oder der zweite Bereich, einen Kantenbereich (26) umfasst, der dazu dient, einen Teil der Öffnung (10) zu definieren.

4. Kappe nach einem der Ansprüche 1 bis 3, bei der der wenigstens erste und zweite Bereich einen kontinuierlichen Ring bilden, der zum Greifen und Umgeben von Hautgewebe (33) ausgelegt ist.

5. Kappe nach Anspruch 4, bei der der kontinuierliche Ring im wesentlichen kreisförmig ist.

6. Kappe nach einem der Ansprüche 1 bis 5, wobei die Kappe (2) wenigstens teilweise aus einem antimikrobiellen Material gebildet ist.

7. Kappe nach einem der Ansprüche 1 bis 5, wobei die Kappe (2) eine Beschichtung aus antimikrobiellen Material umfasst.

8. Kappe nach Anspruch 6 oder Anspruch 7, bei der das antimikrobielle Material eine Trichlorphenol-Verbindung, so wie 2,4,4-Trichlor-2-Hydroxydiphenol, umfasst.

9. Kappe nach einem der Ansprüche 1 bis 8, bei der der wenigstens erste und zweite Bereich nach innen biegbare Bereiche sind.

10. Kappe nach Anspruch 9, bei der die nach innen biegbaren Bereiche hautgreifende Zähne (14) umfassen.

11. Kappe nach einem der Ansprüche 1 bis 10, bei der der wenigstens erste und zweite Bereich einen sechsseitigen Querschnitt (16) haben.

12. Kappe nach Anspruch 11, bei der der sechsseitige Querschnitt (16) einen Innenwinkel (α) umfasst, der eine zusammengedrückte Position der Kappe (2) während des Einsatzes festlegt.

13. Kappe nach einem der Ansprüche 1 bis 12, wobei die Kappe (2) so gestaltet ist, dass sie mit anderen identischen Kappen stapelbar ist.

14. Kappe nach einem der Ansprüche 1 bis 13, bei der der wenigstens erste und zweite Bereich sich federnd deformieren, das Hautgewebe (33) greifen und sich aneinander annähern, so dass eine Wölbung (32) in dem Hautgewebe erzeugt wird.

15. Kappe nach Anspruch 14, wobei die Kappe (2) zum Ausüben eines Vorlancierdrucks ausgestaltet ist, um die Wölbung (32) vor dem Lancieren mit der Vorrichtung (202) zum Durchstechen von Hautgewebe zu halten, und zum Ausüben eines Nachlancierdruckes, um die Wölbung weiter nach dem Durchstechen mit der Vorrichtung (202) zum Durchstechen von Hautgewebe zu halten.

16. Kappe nach einem der vorangehenden Ansprüche, bei der das proximale Ende des Kappengehäuses (4) von der Halterung (6) daran gehindert wird, sich nach außen in eine Ebene im wesentlichen parallel zu einer Ebene, die die Öffnung (10) enthält, zu deformieren.

17. Kappe nach einem der vorangehenden Ansprüche, bei der das proximale Ende des Kappengehäuses (4) frei innerhalb der Halterung (6) ruht.

18. Kappe nach einem der Ansprüche 1 bis 17, bei der die Halterung (6) ein Anschlagstück (34) zum Verhindern der Deformation des Kappengehäuses (4) in eine Richtung im allgemeinen senkrecht zu einer Ebene, die die Öffnung (10) enthält, umfasst.

19. Kappe nach einem der Ansprüche 1 bis 18, bei der die Halterung (6) eine nach innen weisende Ausnehmung zum Aufnehmen des Kappengehäuses (4) und eine nach innen hervorstehende Rippe, die so gestaltet ist, dass sie betrieblich mit der Außenfläche des Kappengehäuses zusammenwirkt, aufweist.

20. Kappe nach einem der Ansprüche 1 bis 19, bei der das Kappengehäuse (4) ein flexibles Kappengehäuse ist.

21. Lanzettenvorrichtung (22), die aufweist:
ein Gehäuse (208, 210);
eine Lanzette (200), die in Bezug auf das Gehäuse bewegbar ist; und
eine Kappe (2) nach einem der Ansprüche 1 bis 19.

22. Kombinierte Lanzetten- und Messvorrichtung zum Durchstechen von Hautgewebe (33), Herausziehen einer Fluidprobe und Messen eines Analyten innerhalb der Fluidprobe, wobei die kombinierte Lanzetten- und Messvorrichtung aufweist:
eine Lanzettenvorrichtung (202) nach Anspruch 21 und eine Messvorrichtung zum Messen des Analyten innerhalb der Fluidprobe.

23. Kombinierte Lanzetten- und Messvorrichtung nach Anspruch 22, bei der eine Fluidsammeleinheit an eine Position benachbart einem Durchstechort transportiert wird, nachdem das Durchstechen vorgenommen ist, so dass die Fluidprobe innerhalb der Messvorrichtung gesammelt und gemessen werden kann, ohne die kombinierte Lanzetten- und Messvorrichtung in Bezug auf die Punktur neu zu positionieren.

24. Kombinierte Lanzetten- und Messvorrichtung nach Anspruch 23, bei der die Fluidsammeleinheit ein Teststreifen ist.

25. Vorrichtung nach einem der Ansprüche 1 bis 24 zur Verwendung beim Sammeln einer Fluidprobe aus Hautgewebe (33).

## Revendications

1. Couvercle (2) destiné à un dispositif autopiqueur de tissu dermique (202), le dispositif autopiqueur de tissu dermique comprenant un logement (208, 210) et une lancette (200) qui est mobile par rapport au logement, le couvercle comprenant :
➢ un corps de couvercle (4) ;
➢ un dispositif de retenue (6) ; et
➢ une ouverture (10) permettant de laisser passer à travers une partie de la lancette (200) :
➢ dans lequel le corps de couvercle (4) présente une extrémité proximale destinée à venir en prise avec le dispositif de retenue (6), et une extrémité distale destinée à venir en prise avec le tissu dermique (33) ;
➢ dans lequel le dispositif de retenue (6) présente une extrémité proximale destinée à venir en prise avec le logement (208, 210), et une extrémité distale destinée à venir en prise avec le corps de couvercle (4) ;
➢ dans lequel l'extrémité distale du corps de couvercle (4) comprend au moins une première partie et une deuxième partie destinées à venir en prise avec le tissu dermique, les au moins une première partie et une deuxième partie pouvant être déformées de manière élastique de telle sorte que lorsqu'il est en service, le couvercle (2) soit en contact avec le tissu dermique (33), et soit poussé vers celui-ci, les au moins une première partie et une deuxième parties se déforment de manière élastique, viennent en prise avec le tissu dermique et se rapprochent l'une de l'autre ;
**caractérisé en ce qu'**une forme de section transversale (16) du couvercle (2) est telle qu'un pivot (A) soit formé sur le corps de couvercle (4) et à l'intérieur du dispositif de retenue (6) de telle sorte que lorsque le couvercle, y compris le corps de couvercle et le dispositif de retenue, sont poussés, en fonctionnement, vers le tissu dermique (33), le corps de couvercle (4) pivote à l'intérieur du dispositif de retenue (6) de telle sorte que la dimension de l'ouverture (10) soit réduite.

2. Couvercle selon la revendication 1, dans lequel les au moins une première partie et une deuxième partie sont ajustées de telle sorte que, lorsque le couvercle (2) entre en contact avec le tissu dermique (33), et est poussé vers celui-ci, les au moins une première partie et une deuxième partie se déforment de manière élastique de telle sorte que la dimension de l'ouverture (10) soit réduite.

3. Couvercle selon la revendication 1 ou la revendication 2, dans lequel l'une au moins de la première partie et de la deuxième partie comprend une partie de bord (26) qui sert à définir une partie de l'ouverture (10).

4. Couvercle selon l'une quelconque des revendications 1 à 3, dans lequel les au moins une première partie et une deuxième partie forment un anneau continu apte à venir en prise avec le tissu dermique (33), et à entourer celui-ci.

5. Couvercle selon la revendication 4, dans lequel l'anneau continu est sensiblement circulaire.

6. Couvercle selon l'une quelconque des revendications 1 à 5, dans lequel le couvercle (2) est réalisé au moins en partie dans un matériau antimicrobien.

7. Couvercle selon l'une quelconque des revendications 1 à 5, dans lequel le couvercle (2) comprend un revêtement de matériau antimicrobien.

8. Couvercle selon la revendication 6 ou la revendication 7, dans lequel le matériau antimicrobien comprend un composé de trichloro-phénol, tel que du 2,4,4, - trichloro - 2 - hydroxy - diphénol.

9. Couvercle selon l'une quelconque des revendications 1 à 8, dans lequel les au moins une première partie et une deuxième partie sont des parties qui peuvent être fléchies vers l'intérieur.

10. Couvercle selon la revendication 9, dans lequel les parties qui peuvent être fléchies vers l'intérieur comprennent des dents de préhension de la peau (14).

11. Couvercle selon l'une quelconque des revendications 1 à 10, dans lequel les au moins une première partie et une deuxième partie comprennent une section cruciforme à six côtés (16).

12. Couvercle selon la revendication 11, dans lequel la section cruciforme à six côtés (16) comprend un angle intérieur (α) qui détermine une position comprimée du couvercle (2) au cours d'une utilisation.

13. Couvercle selon l'une quelconque des revendications 1 à 12, dans lequel le couvercle (2) est configuré de manière à pouvoir être empilé avec d'autres couvercles identiques.

14. Couvercle selon l'une quelconque des revendications 1 à 13, dans lequel les au moins une première partie et une deuxième partie se déforment de manière élastique, viennent en prise avec le tissu dermique (33) et se rapprochent l'une de l'autre de telle sorte qu'un bombement (32) soit créé dans le tissu dermique.

15. Couvercle selon la revendication 14, dans lequel le couvercle (2) est configuré de manière à appliquer une pression de pré-piqûre afin de maintenir le bombement (32) avant la piqûre effectuée à l'aide du dispositif autopiqueur de tissu dermique (202), et de manière à appliquer une pression de post-piqûre afin de maintenir encore le bombement après la piqûre effectuée par le dispositif autopiqueur de tissu dermique.

16. Couvercle selon l'une quelconque des revendications précédentes, dans lequel le dispositif de retenue (6) empêche l'extrémité proximale du corps de couvercle (4) de se déformer vers l'extérieur dans un plan sensiblement parallèle à un plan qui contient l'ouverture (10).

17. Couvercle selon l'une quelconque des revendications précédentes, dans lequel l'extrémité proximale du corps de couvercle (4) repose librement à l'intérieur du dispositif de retenue (6).

18. Couvercle selon l'une quelconque des revendications 1 à 17, dans lequel le dispositif de retenue (6) comprend un arrêt (34) destiné à empêcher une déformation du corps de couvercle (4) dans une direction en général perpendiculaire à un plan qui contient l'ouverture (10).

19. Couvercle selon l'une quelconque des revendications 1 à 18, dans lequel le dispositif de retenue (6) présente un retrait qui fait face vers l'intérieur de manière à recevoir le corps de couvercle (4) et un rebord qui fait saillie vers l'intérieur configuré de manière à coopérer de façon fonctionnelle avec la surface extérieure du corps de couvercle.

20. Couvercle selon l'une quelconque des revendications 1 à 19, dans lequel le corps de couvercle (4) est un corps de couvercle souple.

21. Dispositif de lancette (202) comprenant :
➢ un logement (208, 210) ;
➢ une lancette (200) qui est mobile par rapport au logement ; et
➢ un couvercle (2)selon l'une quelconque des revendications 1 à 19.

22. Lancette et dispositif de dosage combinés destinés à piquer un tissu dermique (33), à extraire un échantillon de fluide et à mesurer une substance à analyser à l'intérieur dudit échantillon de fluide, la lancette et le dispositif de dosage combinés comprenant :
un dispositif de lancette (202) selon la revendication 21 et un dispositif de dosage destiné à mesurer la substance à analyser à l'intérieur de l'échantillon de fluide.

23. Lancette et dispositif de dosage combinés selon la revendication 22, dans lesquels un dispositif de collecte de fluide est prévu à une position adjacente à une position de piqûre une fois qu'une piqûre a été exécutée de telle sorte que l'échantillon de fluide puisse être collecté et mesuré à l'intérieur du dispositif de dosage sans avoir à repositionner la lancette et le dispositif de dosage combinés par rapport à la piqûre.

24. Lancette et dispositif de dosage combinés selon la revendication 23, dans lesquels le dispositif de collecte de fluide est une bandelette réactive.

25. Dispositif selon l'une quelconque des revendications 1 à 24, destiné à être utilisé de manière à collecter un échantillon de fluide en provenance d'un tissu dermique (33).
